Europäisches Patentamt

European Patent Office

Office européen des brevets

(19)

(11) Publication number: **0 445 861 A1**

## EUROPEAN PATENT APPLICATION

(12)

(21) Application number: 91200373.8

(22) Date of filing: 21.02.91

(51) Int. Cl.5: **G01N 33/22**

(30) Priority: 24.02.90 NL 9000449

(43) Date of publication of application:
11.09.91 Bulletin 91/37

(84) Designated Contracting States:
AT BE CH DE DK ES FR GB GR IT LI NL SE

(71) Applicant: N.V. NEDERLANDSE GASUNIE
Postbus 19
NL-9700 MA Groningen(NL)

(72) Inventor: Dijkstra, Kees
Troelstralaan 63 D
NL-9722 JE Groningen(NL)

(74) Representative: Bleukx, Lucas Lodewijk Maria,
Ir. et al
OCTROOIBUREAU DSM Postbus 9
NL-6160 MA Geleen(NL)

(54) **Method and device for determination of the Wobbe index.**

(57) Method for determination of the Wobbe index of a gas mixture, wherein the gas mixture is supplied from a supply section (20) to a first buffer chamber (23) and via at least one flow opening to a mixing chamber (30), an oxidation gas is supplied from a supply section (10) to a second buffer chamber (13) and via at least one flow opening to the mixing chamber (30), a second gas mixture is formed in the mixing chamber and this second gas mixture is burned in a combustion chamber, the supply from one supply section to one of the buffer chambers is realized via a reducing valve (12) (22) so that a constant pressure is maintained in said buffer chamber, and the temperature of the second gas mixture before combustion and the temperature of the gas formed on combustion is measured, and the Wobbe index is determined from the temperatures thus measured. The two buffer chambers are connected via a chamber (31) that is connected with the atmosphere via a flow orifice (32) and the gas is supplied to the other buffer chamber at a constant flow rate, which flow rate is slightly greater than the normal flow rate from this buffer chamber to the mixing chamber.

Fig. 1

The invention relates to a method for determination of the Wobbe index of a gas mixture, the gas mixture being supplied from a supply section to a first buffer chamber and being fed, via at least one flow opening, from this first buffer chamber to a mixing chamber, an oxidation gas being supplied from a supply section to a second buffer chamber and being fed, via at least one flow opening, from said second buffer chamber to the mixing chamber, a second gas mixture being formed in the mixing chamber and this second gas mixture being burned in a combustion chamber, the supply from one supply section to one of the buffer chambers being realized via a pressure-reducing valve so that a constant pressure is maintained in said buffer chamber and the temperature of the second gas mixture before combustion and that of the gas formed on combustion being measured and the Wobbe index being determined from the temperatures thus measured.

Such a method is known from the commercially marketed Union Wobbe meter.

The disadvantage of this known method is that the pressure in the two buffer chambers is controlled by means of pressure-reducing valves. This is an absolute means of control which, under normal conditions, ensures a constant mixing ratio in the mixing chamber. Minor deviations in one of the pressure-reducing valves can, however, disorder the system and give rise to serious errors.

The aim of the invention is to supply a method of the kind mentioned above, with which the aforementioned disadvantages are avoided.

This aim is achieved according to the invention because the two buffer chambers are connected via a chamber that is connected with the atmosphere via a flow orifice and the gas is supplied to the other buffer chamber at a constant flow rate, which flow rate is slightly greater than the normal flow rate from this buffer chamber to the mixing chamber.

Because only the pressure in one of the buffer chambers is controlled by means of a pressure-reducing valve, the pressure in the other buffer chamber automatically becomes the same as that in the first buffer chamber via the communicating chamber. Variations in the pressure of the first chamber automatically lead to an adjustment of the pressure in the second buffer chamber, the amount of gas flowing to the mixing chamber being automatically adjusted to the same mixing ratio. The excess gas supplied to the second buffer chamber is discharged, while the supplied excess provides automatic compensation.

Preferably, the second buffer chamber is chosen as buffer chamber with a constantly controlled pressure.

By keeping the oxidation gas at a constant pressure a less sensitive system is obtained because of the supplied amounts of gas this flow usually has the greatest volume.

Other characteristics and advantages will become evident from the following description, in which reference is made to the attached drawings, where:

Figure 1 schematically represents a Wobbe meter suitable for determining the Wobbe index according to the invention,

Figure 2 schematically represents an embodiment that is changed relative to Figure 1 and

Figure 3 schematically represents an embodiment of the burner that is changed relative to Figure 2.

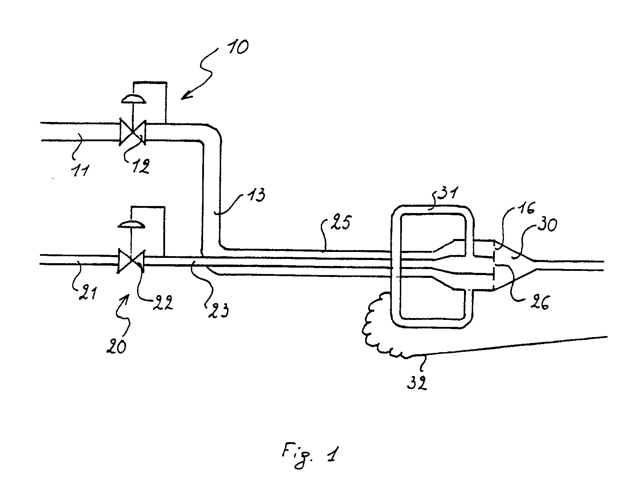

The device as shown in Figure 1 comprises a supply section 10 for the oxidation gas and a supply section 20 for the gas mixture of which the Wobbe index is to be determined. The oxidation gas may be air, but use may also be made of any other gas by which the gas mixture can be oxidized, such as pure oxygen, oxygen-enriched air, peroxides, etc.

The gas mixture may be natural gas, but in principle any other gas or gas mixture suitable for combustion purposes can be examined with the aid of the method according to the invention.

Oxidation gas supply section 10 comprises a supply line 11, which on one side is connected with an oxidation gas source supplying an oxidation gas at substantially constant pressure, for example atmospheric air. On the other side supply line 11 is connected with a pressure control valve 12, via which the oxidation gas can flow into a pipe section or buffer chamber 13. Pressure control valve 12 is used to control the gas pressure in pipe section or buffer chamber 13 at a constant level.

Gas mixture supply section 20 comprises a supply line 21, which on one side is connected with a source of the gas mixture to be measured, and on the other with a flow-control valve 22, through which the gas mixture can flow to a pipe section or buffer chamber 23. The setting of the control valve 22 will be discussed later on. The valve concerned is of a type that is commonly known and is marketed by, for example, Brooks under the name flow-controller.

Pipe sections 13 and 23 are at least in part parallel and in heat-exchanging contact, for instance via a coaxial section 25. If necessary, an electrical heating device may be provided around the coaxial section, which can be used to heat the gas mixture and the oxidation gas to the same temperature. Pipe section 13 ends in a mixing chamber 30 via a flow opening or a number of flow openings 16. Pipe section 23 also ends in the mixing chamber 30 via a flow opening 26.

Pipe section 13 and pipe section 23 are connected to one another via a pipe 31, so that the same pressure is maintained in the two pipe sections. Pipe 31 is connected to the atmosphere via a capillary duct 32 set about halfway between the two pipe sections 13 and 23. Optionally, a burner is installed in the capillary duct.

The mixing chamber 30 is in turn connected with a combustion chamber, not shown in Figure 1, where the gas mixture formed in the mixing chamber 30 is burned. Preferably, the mixing chamber is fitted with a thermometer with which the temperature of the gas mixture formed in the mixing chamber is measured. Optionally, it is possible to measure the temperature already before the mixing chamber, for example the temperature of the oxidation gas in pipe section 13. This is possible because the oxidation gas constitutes the largest volume of the gas mixture in the mixing chamber 30 and because the two gas flows are brought to and maintained at virtually the same temperature.

A thermometer is also installed immediately after the burner, which is used to determine the temperature of the flue gas formed in the combustion. A comparison of the temperature of the supplied gas mixture and the temperature of the flue gas gives an indication of the Wobbe index. If the temperature is determined electrically, it may be translated directly into the Wobbe index, for example via a bridge circuit. Preferably, the temperature measuring device is therefore fitted with a resistance wire, for example a nickel wire, one end of which is suspended in the mixing chamber and the other after the burner, which can thus be directly incorporated in a bridge circuit.

However, it is also possible to use the Wobbe meter as described in combination with a probe as described in the Netherlands patent applications 8901660 and 8802336. Other elements known from these patent applications may also be used in the Wobbe meter currently discussed.

The dimensions of openings 16 and 26 are chosen so that the Reynolds numbers of both openings are in principle the same, with due allowance for the different volumes flowing through the openings and the flow properties. The flow rate of the oxidation gas is chosen so that there is always an excess of oxidation gas in the mixing chamber, so that complete combustion is realized in the burner.

The device according to Figure 1 operates as follows. Via line 11, valve 12 and pipe section 13 air is supplied to opening 16 at a constant pressure. A portion of this air flows into mixing chamber 30 via opening 16, while another portion can optionally flow away via pipe 31 and the capillary pipe 32.

Via line 21, valve 22 and pipe section 23 a gas mixture of which the Wobbe index is to be determined is supplied to opening 26. Because of pipe 31 the pressure of the gas mixture in pipe section 23 is the same as the pressure of the air in pipe section 25. In addition, the setting of valve 22 is chosen so that the amount of gas that flows through valve 22 is greater than the amount of gas that flows into mixing chamber 30 via opening 26 at the pressure determined by the setting of valve 12. The excess gas supplied to pipe section 23 is discharged via pipe 31 and the capillary pipe 32.

Because the pressure in pipe sections 13 and 23 is the same on account of pipe 31 - and in principle it is maintained at a constant level via pressure control valve 12 - the amounts of gas and air that flow through openings 26 and 16, respectively, will always be the same and a constant gas/air mixing ratio will be created in the mixing chamber, which is a prerequisite for a reproducible determination of the Wobbe index.

If the pressure determined by the pressure control valve 12 should for some reason be set at a different value, this will not affect the mixing ratio in the mixing chamber 30 because a rise in pressure means that more gas will flow into mixing chamber 30. If the Reynolds numbers of openings 16 and 26 are the same, the amount of air that flows into the mixing chamber will be modified accordingly. The mixing ratio remains the same but the amount of gas that is discharged via the capillary pipe decreases. If the pressure determined by pressure control valve 12 decreases, less air will flow into mixing chamber 30, but because the pressure in pipe section 23 has also decreased, less gas will also flow into mixing chamber 30. Because the amount of gas remains constant, a larger amount of gas is discharged via the capillary pipe 32.

Optionally, the gas mixture discharged via the capillary pipe 32 can be burned. On the other hand, it is also possible to measure the discharged amount of gas and to adjust the setting of valve 22 dependent on this amount. In this respect it is important that the amount of gas supplied via valve 22 is always an excess amount of gas. In principle, it is also possible to reverse the roles of the gas and the air, in the sense that the gas is supplied at a constant pressure while the air is supplied in a constant volume. However, this situation is somewhat less advantageous when use is made of the principle of temperature measurement because the supplied air has the largest volume.

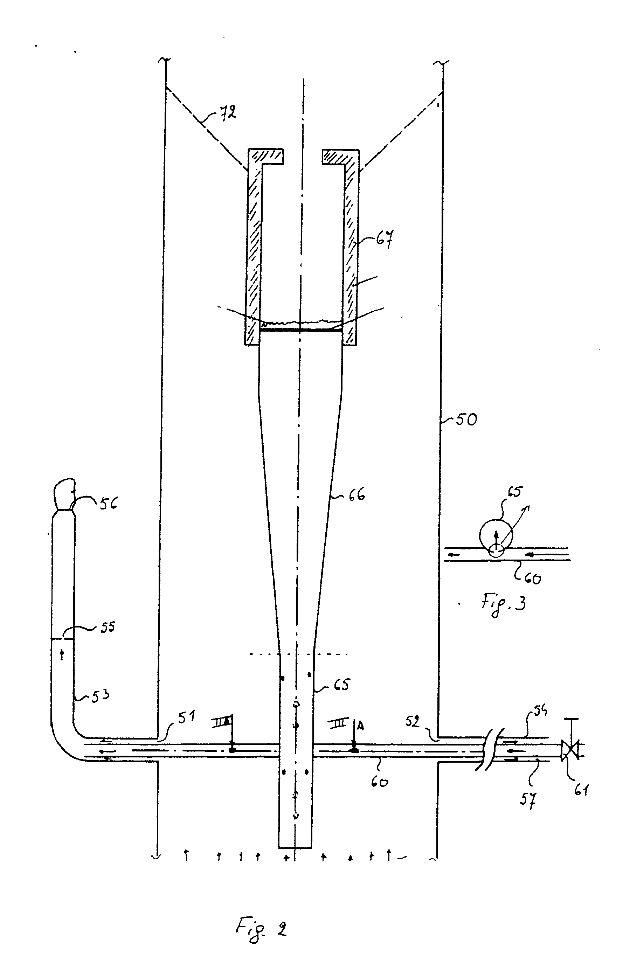

Figure 2 shows a second embodiment of a Wobbe meter according to the invention. The device illustrated here consists essentially of a vertically placed cylindrical pipe 50. The bottom end of this pipe, which is not illustrated, is connected with a fan which blows air into pipe 50. At a certain height are two diametrically opposed connection openings 51 and 52, to which pipe sections 53 and 54, respectively, are connected. Pipe section 53 is L-shaped, with one leg projecting upwards. At the top, it is provided with a free outlet, in which, optionally, provisions for a pilot light may be fitted. Pipe section 53 is also fitted with a flow orifice 55. Pipe section 54 extends horizontally and is provided with a free opening 57.

Diametrically through pipe 50 and inside pipe sections 53 and 54 extends a pipe 60, which on one side ends in pipe section 53 and on the other side is connected with a supply of gas via a flow-controller 61.

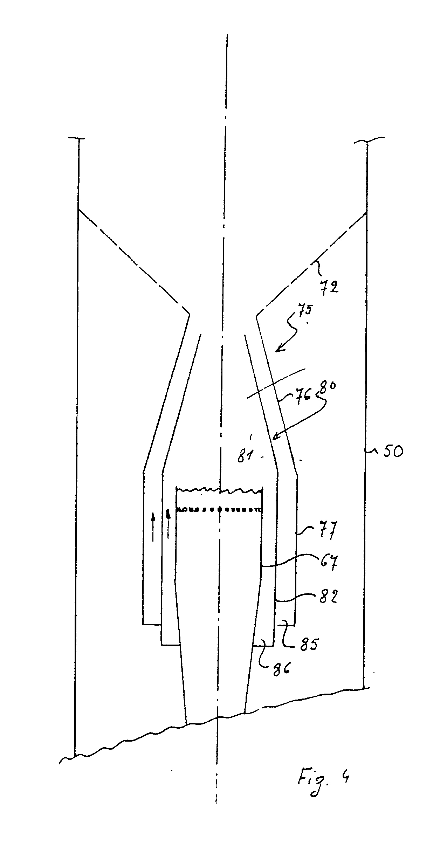

In the middle of pipe 50 a pipe section 65 is provided perpendicularly to pipe 60, in a manner shown in greater detail in Figure 3. Pipe section 65 has a conically widening section 66, which finally changes into a cylindrical section 67 made of an insulating material. A burner is formed in the bottom end of section 67. A number of openings are formed in pipe section 65, via which the interior of pipe section 65 is connected with pipe 50. This enables air supplied to pipe 50 to flow to pipe section 65. As shown in Figure 3, pipe 60 is also connected with pipe section 65 via an opening 70. In this manner gas supplied to pipe 60 can be supplied to pipe section 65.

The gas mixture formed in pipe section 65 is then burned in section 67 and can escape as flue gas via the top end of pipe 50. An electric resistance wire may be fitted in pipe section 65 and above burner section 67, which can be used to determine the temperature of the gases flowing through that section.

Burner section 67 is connected with the inside wall of pipe 50 via a partition 72. A number of openings are provided in this partition 72 so that air supplied by the fan can mix with the flue gases. Because the pressure in pipe 50 is constant and the pressure above partition 72 is also constant, i.e. atmospheric pressure, the amount of air flowing through partition 72 will be constant in proportion to the other gas and air flows. The mixture of flue gas and air therefore has a constant mixing ratio, which means that the temperature of this gas mixture is proportionally lower, but can still be used to determine the Wobbe index.

The opening between pipe section 60 and pipe section 65 is a calibrated opening so that a controlled amount of gas determined by the pressure set in pipe 50 flows through it. Preferably, the same Reynolds number is chosen for both types of openings, in accordance with the embodiment shown in Figure 1.

The operation of the device according to Figure 2 is in principle the same as that of Figure 1, the excess amount of supplied gas being discharged via pipe section 53 and the excess amount of supplied air being discharged via the same pipe section 53 and the annular opening between pipe sections 54 and 60. The opening 57 also enables heat exchange with the gas flow in the pipe, etc.

Figure 4 shows a modified embodiment of the burner pipe 67. Here the partition 72 is not fitted between the burner pipe 67 and the inside wall of pipe 50 but between the inside wall of pipe 50 and wall 75. Wall 75 consists of a conical top part 76 and a cylindrical bottom part 77 that extends coaxially over the burner pipe 67 so that an opening with an annular cross section is created between the burner pipe and section 77.

A second wall 80, of the same shape as wall 75, with a conical top part 81 and a cylindrical bottom part 82, is provided coaxially with respect to wall 75. In this way two openings with annular cross sections are created, an opening 85, through which air flows that is used to cool wall 80, and an opening 86, through which air flows that is mixed with the flue gas, whose temperature it reduces in a known manner, so that the measurement can be carried out with greater accuracy. The temperature of the flue gas is measured in the area above the partition 72. This system prevents excessive heat exchange with fixed parts in the burner.

## Claims

1. Method for determination of the Wobbe index of a gas mixture, the gas mixture being supplied from a supply section to a first buffer chamber and being supplied via at least one flow opening from said first buffer chamber to a mixing chamber, an oxidation gas being supplied from a supply section to a second buffer chamber and being supplied via at least one flow opening from the second buffer chamber to the mixing chamber, a second gas mixture being formed in the mixing chamber and this second gas mixture being burned in a combustion chamber, the supply from one supply section to one of the buffer chambers being realized via a reducing valve so that a constant pressure is maintained in said buffer chamber, and the temperature of the second gas mixture before combustion and the temperature of the flue gas formed in the combustion being measured, and the Wobbe index being determined from the temperatures thus measured, characterized in that the two buffer chambers are connected via a chamber that is connected with the atmosphere via a flow orifice and that the gas is supplied to the other buffer chamber at a constant flow rate, which flow rate is slightly greater than the normal flow rate from this buffer chamber to the mixing chamber.

2. Method according to claim 1, characterized in that the buffer chamber with the constantly controlled pressure is the second buffer chamber.

3. Method according to claim 1 or 2, characterized in that the flue gas is mixed with an amount of oxidation gas supplied from the second buffer chamber before the temperature is measured.

4. Method according to any one of claims 1-3, characterized in that the connection opening(s) between the first buffer chamber and the mixing chamber and the connection opening(s) between the second buffer chamber and the mixing chamber have the same Reynolds number.

5. Method according to any one of the preceding claims, characterized in that the amount of gas that escapes via the flow orifice is used as a signal for setting the flow rate of the gas flow with the constant flow rate.

Fig. 1

EP 0 445 861 A1

Fig. 3

Fig. 2

7

Fig. 4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | DE-B-1 027 912 (H.F. REINEKE) <br> * Figure 1 * <br> --- | 1 | G 01 N 33/22 |
| A | DE-C-9 002 79 (O. DOMMER) <br> * Figure 1 * <br> --- | 1 | |
| A | EP-A-0 326 494 (R. GUILLET) <br> * Front page * <br> --- | 1 | |
| A | EP-A-0 323 658 (K. DIJKSTRA) <br> * Front page * <br> ----- | 1 | |

| | | | TECHNICAL FIELDS SEARCHED (Int. Cl.5) |
|---|---|---|---|
| | | | G 01 N |

The present search report has been drawn up for all claims

| Place of search | Date of completion of search | Examiner |
|---|---|---|
| The Hague | 21 June 91 | DUCHATELLIER M.A. |